# FASCICULE DE BREVET EUROPEEN

(11) **EP 1 438 291 B1**
(45) Date de publication et mention de la délivrance du brevet: **07.09.2005**
(21) Numéro de dépôt: 02795311.6
(22) Date de dépôt: 14.10.2002
(51) Int. Cl.: C07D 211/70, C07D 401/04, A61K 31/444, A61P 29/00

(54) **ARALKYL-TETRAHYDRO-PYRIDINES, LEUR PREPARATION ET COMPOSITIONS PHARMACEUTIQUES LES CONTENANT**
ARALKYLTETRAHYDROPYRIDINE, DEREN HERSTELLUNG UND PHARMAZEUTISCHE ZUSAMMENSETZUNGEN, DIE DIESE ENTHALTEN
ARALKYL-TETRAHYDRO-PYRIDINES, THEIR PREPARATION AND PHARMACEUTICAL COMPOSITIONS CONTAINING SAME

(30) Priorité: 18.10.2001 FR 0113469
(43) Date de publication de la demande: 21.07.2004
(73) Titulaire: Sanofi-Aventis, 75013 Paris (FR)
(72) Inventeur: BARONI, Marco, I-20010 Vanzago (IT); BOURRIE, Bernard, F-34980 Saint Gely du Fesc (FR); CARDAMONE, Rosanna, I-22100 Como (IT); CASELLAS, Pierre, F-34000 Montpellier (FR)
(74) Mandataire: Varady, Peter
(86) Numéro de dépôt international: PCT/FR2002/003508
(87) Numéro de publication internationale: WO 2003/033466

(56) Documents cités:
- US-A- 5 281 606
- BORRI ET AL.: "The neuroprotective agent SR57746A abrogats experimental autoimmune encephalomyelitis and impairs associated blood-brain barrier disruption: Implications for multiple sclerosis treatment" PROC. NATL. ACAD. SCI. USA, vol. 96, no. 22, 1999, pages 12855-9, XP002205352 cité dans la demande

## Description

La présente invention concerne de nouvelles aralkyl-tétrahydro-pyridines, les compositions pharmaceutiques les contenant, un procédé pour leur préparation et des intermédiaires de synthèse dans ce procédé.

EP 0 458 697 décrit des dérivés de la naphtylalkyl-tétrahydro-pyridine ayant une activité modulatrice de la motricité intestinale.

Bourrié et al. (Proc. Natl. Acad. Sci. 1999, 96(22):12855-12859) ont décrit l'activité d'un composé dénommé SR 57746 (1-(2-napht-2-yléthyl)-4-(3-trifluorométhyl)-1,2,3,6-tétrahydropyridine) dans un modèle d'encéphalomyélite auto-immune expérimentale (EAE) et dans la modulation du TNF-alpha (de l'anglais Tumour Necrosis Factor).

Il a été maintenant trouvé que certaines tétrahydro-pyridines, substituées par un radical aralkyle partiellement saturé, possèdent une puissante activité vis-à-vis de la modulation du TNF-alpha.

Le TNF-alpha est une cytokine qui a récemment suscité de l'intérêt en tant que médiateur de l'immunité, de l'inflammation, de la prolifération cellulaire, de la fibrose etc. Ce médiateur est copieusement présent dans le tissu synovial enflammé et exerce un rôle important dans la pathogenèse de l'auto-immunité (Annu. Rep. Med. Chem., 1997, 32:241-250).

Ainsi, la présente invention concerne, selon un de ses aspects, des aralkyl-tétrahydro-pyridines de formule (I): 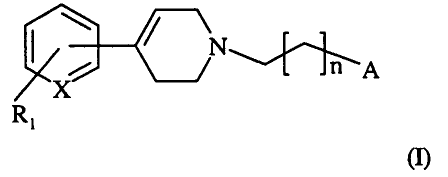 dans laquelle
- X: représente N ou CH;
- R₁: représente un atome d'hydrogène ou d'halogène ou un groupe CF₃;
- n: est un entier de 1 à 5 ;
- A: représente un groupe de formule de (a) à (d): 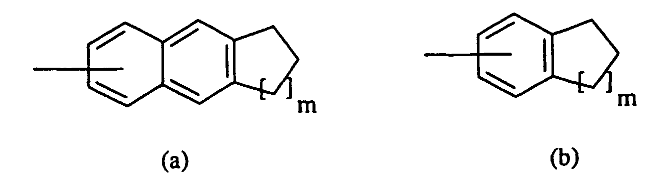 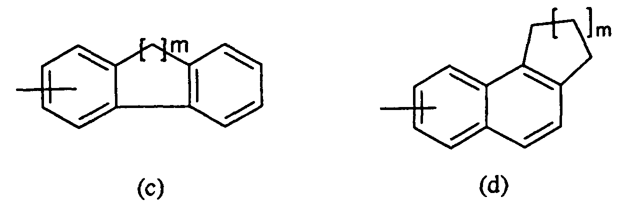 dans lesquelles m est 1 ou 2 ;
ainsi que leurs sels ou solvates et leurs N-oxydes.

Dans la présente description, le terme "halogène" désigne un atome choisi parmi le chlore, le brome, le iode et le fluor.

Des composés préférés sont ceux où n est 1.

D'autres composés préférés sont ceux où R₁ est un groupe CF₃.

D'autres composés préférés sont ceux où X est CH et R₁ est dans la position 3 du benzène.

D'autres composés préférés sont ceux où X est CH et R₁ est un groupe CF₃.

D'autres composés préférés sont ceux où X est N et la pyridine est substituée dans les positions 2,6.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Selon la présente invention, les composés de formule (I) peuvent exister comme dérivés N-oxydes, notamment ils peuvent porter un groupe N-oxyde sur la tétrahydro-pyridine.

Les composés de formule (I) peuvent être synthétisés par un procédé qui prévoit
(a) de faire réagir le composé de formule (II): 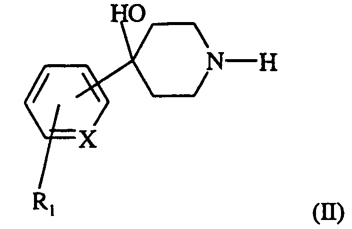 dans laquelle R₁ est défini comme précédemment, avec l'acide de formule (III) ou un de ses dérivés fonctionnels: 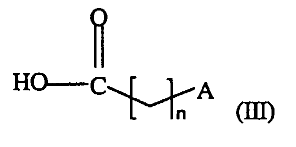 dans laquelle n et A sont tels que définis ci-dessus,
(b) de réduire le groupe carbonyle du composé de formule (IV) ainsi obtenu: 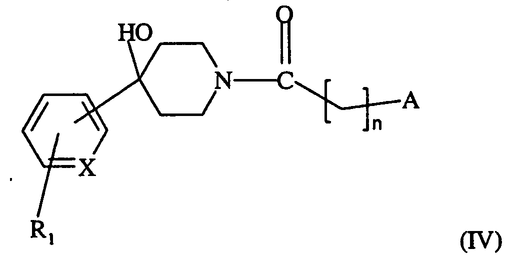
(c) de déshydrater le pipéridinol intermédiaire de formule (V) ainsi obtenu 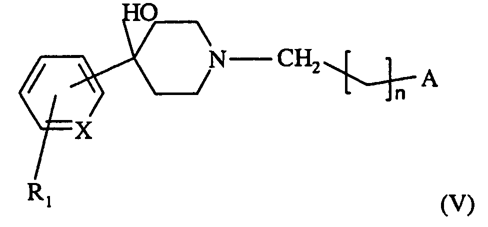
(d) d'isoler le composé de formule (I) ainsi obtenu et, éventuellement le transformer en l'un de ses sels ou solvates ou dans ses dérivés N-oxyde.

La réaction de l'étape (a) peut être convenablement conduite dans un solvant organique à une température comprise entre -10°C et la température de reflux du mélange réactionnel.

Il peut être préférable de réaliser la réaction à froid lorsqu'elle est exothermique, comme dans le cas où on utilise le chlorure en tant que dérivé fonctionnel de l'acide de formule (III).

Comme composé de formule (III), on peut utiliser soit l'acide libre, éventuellement activé (par exemple avec le BOP, à savoir le tri(diméthylamino)benzotriazol-1-yloxyphosphonium hexafluorophosphate), soit l'un de ses dérivés fonctionnels tel que, par exemple, un anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

Comme solvant de réaction, on utilise de préférence un solvant halogéné tel que le chlorure de méthylène, le dichloroéthane, le 1,1,1-trichloroéthane, le chloroforme et similaires, mais aussi d'autres solvants organiques compatibles avec les réactifs employés, par exemple le dioxane, le tétrahydrofurane ou un hydrocarbure tel que l'hexane, peuvent être également employés.

La réaction peut être convenablement conduite en présence d'un accepteur de protons, par exemple d'un carbonate alcalin ou d'une amine tertiaire telle que la triéthylamine.

La réduction de l'étape (b) peut être convenablement réalisée par des agents de réduction appropriés tels que les complexes du borane, par exemple le - diméthylsulfure de borane ([CH₃]₂S-BH₃), les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofurane (THF), le dioxane ou le 1,2-diméthoxyéthane.

Selon un mode opérationnel préférentiel, on opère avec le diméthylsulfure de borane utilisé en excès par rapport au composé (II) de départ, à la température de reflux éventuellement sous atmosphère inerte. La réduction est normalement terminée après quelques heures.

La déshydratation de l'étape (c) est aisément conduite par exemple en utilisant un mélange acide acétique/acide sulfurique, à une température comprise entre la température ambiante et la température de reflux du solvant utilisé.

Selon une méthode préférée la réaction de l'étape (c) est conduite dans un mélange acide acétique/acide sulfurique dans un rapport de 3/1 en volume, en chauffant à la température d'environ 80-100°C pendant 1 à 3 heures.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou le 2-propanol, un éther comme le 1,2-diméthoxyéthane, l'acétate d'éthyle, l'acétone ou un hydrocarbure comme l'hexane.

Le composé de formule (I) obtenu est éventuellement transformé en un de ses dérivés N-oxyde.

Les composés de formule (I) portant un groupe N-oxyde sur l'atome d'azote de la tétrahydro-pyridine peuvent être préparés par oxydation du composé de formule (I) correspondant. Dans ce cas, le composé de formule (I) est soumis à une réaction d'oxydation selon les méthodes conventionnelles, par exemple à une réaction avec de l'acide m-chloro-perbenzoïque dans un solvant convenable et isolé selon les techniques usuelles bien connues à l'homme du métier.

Les composés de formule (I) peuvent également être préparés par une réaction de condensation à partir d'une tétrahydropyridine de formule (VI): 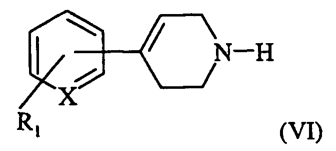 dans laquelle X et R₁ sont tels que définis ci-dessus, avec un composé de formule (VII): 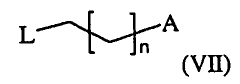 dans laquelle n et A sont tels que définis précédemment, et L est un groupe partant, isolement du composé de formule (I) ainsi obtenu et transformation éventuelle en un de ses sels ou solvates.

Comme groupe partant « L » on peut par exemple utiliser un groupe halogène, ou tout autre groupe apte à la condensation avec le composé de formule (VI).

La réaction de condensation est conduite en mélangeant les composés de départ (VI) et (VII) dans un solvant organique tel qu'un alcool tel que par exemple le méthanol ou le butanol, en présence d'une base telle que par exemple des carbonate alcalins, à une température comprise entre la température ambiante et le reflux du solvant choisi, selon les méthodes conventionnelles.

Les composés de départ de formule (II) sont connus ou bien ils peuvent être préparés de façon analogue aux composés connus.

Les composés de formule (III°) 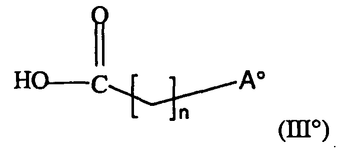 dans laquelle A° est un groupe de formule (a), (c) ou (d) comme définis ci-dessus, n est un entier de 1 à 5 et m est 1 ou 2, ainsi que ses sels ou solvates sont des composés nouveaux et constituent un aspect ultérieur de la présente invention.

Lorsque A est un groupe (a), ces nouveaux composés sont représentés par la formule (III') 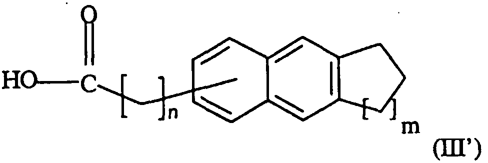 où n et m sont tels que définis ci-dessus, et peuvent être synthétisés selon par un procédé qui prévoit:
(i) de faire réagir le composé (VIII) ci-dessous 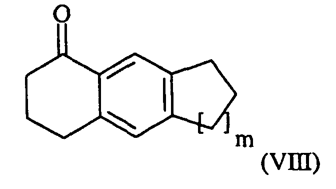 où m est 1 ou 2, avec un dialkylcarbonate en présence d'une base forte telle qu'un hydrure d'un métal alcalin,
(ii) de faire réagir le composé de formule (IX) ainsi obtenu 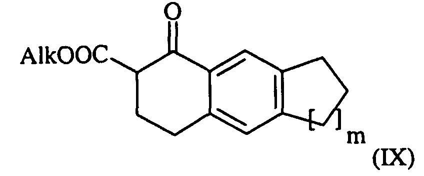 avec un dérivé Hal-(CH₂)ₙ-COOAlk dans laquelle n est tel que défini précédemment, Alk est un groupe alkyle et Hal est un atome d'halogène, en présence d'une base forte telle qu'un hydrure alcalin,
(iii) d'hydrolyser et décarboxyler le composé (X) ainsi obtenu 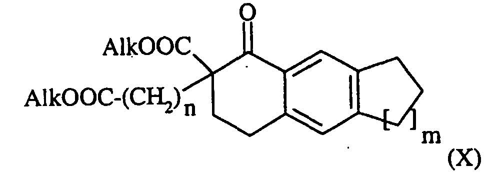 par chauffage en présence d'une base, telle que l'hydroxyde de sodium,
(iv) de réduire la cétone de formule (XI) ainsi obtenue 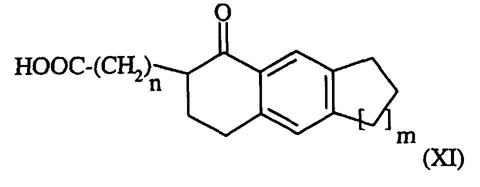
(v) d'aromatiser le composé de formule de formule (XII) ainsi obtenu 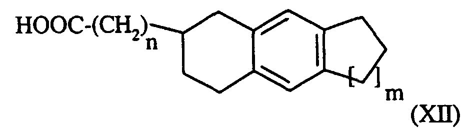 par exemple par oxydation avec du Pd/C à haute température, dans un solvant tel que la décaline, en obtenant le composé de formule (III') qui est isolé selon les méthodes conventionnelles et éventuellement transformé en l'un de ses sels.

Lorsque A est un groupe (c), ces nouveaux composés sont représentés par la formule (III") 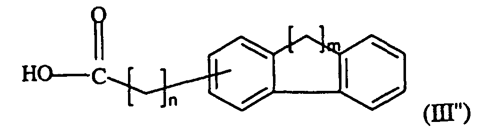 où n et m sont tels que définis ci-dessus.
Lorsque n est 0, ces composés peuvent être synthétisés selon par un procédé qui prévoit:
(vi) de faire réagir le composé de formule (XIII) 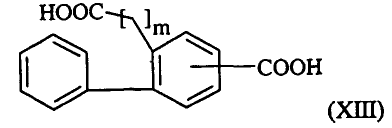 avec de l'acide polyphosphorique (PPA) et réduire le dérivé 9-oxofluorène ainsi obtenu en obtenant le composé de formule (III") où n est 0; et, lorsque n est 1,
(vii) d'estérifier et réduire l'acide fluorèn-carboxylique (XIV) ainsi obtenu: 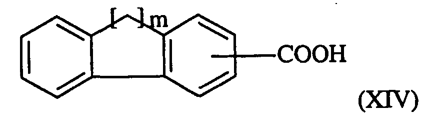 par exemple par réaction avec du méthanol en présence d'acide chlorhydrique suivie par réduction avec du LiAlH₄
(viii) de transformer l'alcohol de formule (XV) ainsi obtenu 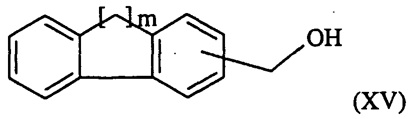 en cyano-dérivé, par exemple par préparation du chlorure correspondant et réaction avec du KCN;
(ix) et d'hydrolyser ce dérivé cyano de formule (XVI) 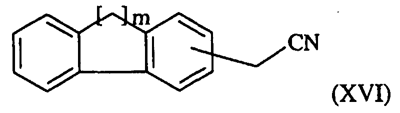 par exemple en milieu acide, en obtenant le composé de formule (III") où n est 1, qui est isolé selon les méthodes conventionnelles et éventuellement transformé en l'un de ses sels.

Si l'on souhaite préparer les composés de formule (III") où n est >1, il suffit de réproduire les passages (vii)-(ix), bien que d'autres procédés de synthèse connus peuvent être utilisés pour allonger la chaîne alkylique. Lorsque A est un groupe (d), ces nouveaux composés sont représentés par la formule (III''') 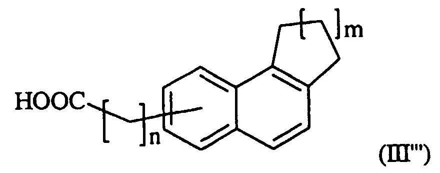 où n et m sont tels que définis ci-dessus.
Les composés de formule (III''') où n est 0 ou 1, peuvent être synthétisés selon par un procédé qui prévoit:
(x) de faire réagir le composé de formule (XVII) 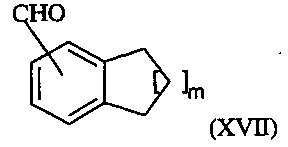 avec du (1-cyano-3,3-diéthoxy-propyl)-diéthylphosphonate;
(xi) de cycliser le composé de formule (XVIII) ainsi obtenu: 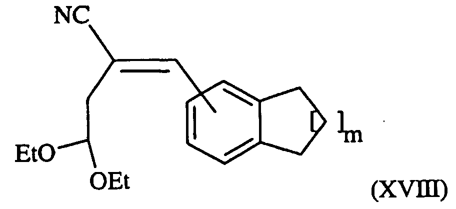 par exemple en présence de SnCl₄, et
(xii) soit d'hydroliser le cyano dérivé de formule (XIX) 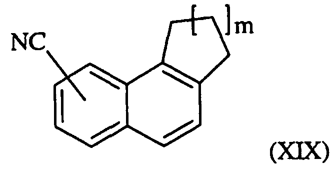 en obtenant le composé de formule (III''') où n est 0,
(xiii) soit, lorsque n est 1, de transformer le dérivé de formule (XIX) en cétone de formule (XX) 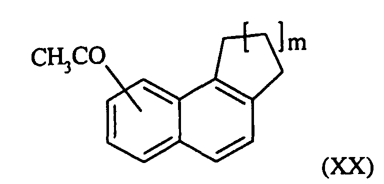 par exemple par réaction de Grignard avec du CH₃MgI; et
(xiv) de transformer la cétone (XX) en l'acide correspondant de formule (III''') où n est 1.

Si l'on souhaite préparer les composés de formule (III''') où n est >1, il suffit de réproduire les passages (vii)-(ix) ci-dessus à partir de l'acide de formule (III''') tel qu'obtenu par le passage (xiv), ou d'utiliser d'autres procédés de synthèse connus pour allonger la chaîne alkylique.

Lorsque la cyclisation du passage (xi) est conduite à partir d'un bétaaldéhyde, il est possible d'obtenir deux dérivés de formule (XIX), car la formation du noyau aromatique peut s'achever par cyclisation sur l'une des deux positions adjacentes. Dans ce cas, il convient de séparer les isomères ainsi formés, par exemple au moyen d'une chromatographie sur colonne de gel de silice, et de poursuivre en utilisant l'isomère souhaité.

Les réactions décrites ci-dessus sont, en général, bien connues à l'homme du métier. Des exemples en détail de tels procédés sont en tous cas rapportés dans la partie expérimentale.

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055:B5, Sigma, St Louis, Mo).

Les produits à tester sont administrés par voie orale à des groupes de 5 souris Balb/c femelles (Charles River, France) âgées de 7 à 8 semaines. Une heure après, le LPS est administré par voie intraveineuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, des composés représentatifs de l'invention se sont montrés très actifs, en inhibant la synthèse du TNF-α même à doses très faibles.

Grâce à cette activité et à leur faible toxicité, les composés de formule (I) et ses sels ou solvates peuvent bien être utilisés dans le traitement des maladies liées à des troubles immunitaires et inflammatoires ou comme analgésiques. Notamment les composés de formule (I) peuvent être utilisés pour traiter l'athérosclérose, les maladies auto-immunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations.

Les composés de formule (I) et leurs sels et solvates pharmaceutiquement acceptables sont de préférence administrés par voie orale.

Dans les compositions pharmaceutiques de la présente invention par voie orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,001 à 100 mg, mieux de 0,01 à 50 mg, de préférence de 0,1 à 20 mg de principe actif, avantageusement de 0,5 à 10 mg.

Selon un autre de ses aspects, la présente invention concerne une association comprenant un composé de formule (I) ou l'un de ses sels ou solvates pharmaceutiquement acceptables, et au moins un composé choisi parmi les agents immunosuppresseurs, tel que l'interféron bêta-1b; l'hormone adrénocorticotrope; les glucocorticoïdes tels que la prédnisone ou la méthylprednisolone; les inhibiteurs de l'interleukine-1.

Plus particulièrement, l'invention concerne une association comprenant un composé de formule (I), ou l'un de ses sels ou solvates pharmaceutiquement acceptables et au moins un composé choisi parmi le roquinimex (1,2-dihydro-4-hydroxy-N,1-diméthyl-2-oxo-3-quinolinecarboxanilide), le myloran (produit de la société Autoimmune contenant de la myéline bovine), l'antegren (anticorps humain monoclonal des sociétés Elan/Athena Neurosciences) l'interféron bêta-lb recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-aminopyridine).

Selon un autre de ses aspects, l'invention concerne une méthode de traitement des maladies liées à des troubles immunitaires et inflammatoires ainsi que dans le traitement de la douleur, notamment l'athérosclérose, les maladies auto-immunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I) ou de l'un de ses sels ou solvates pharmaceutiquement acceptables, seul ou en association avec d'autres principes actifs.

Les exemples qui suivent illustrent l'invention.

### PREPARATION 1

### 1,2,3,6,7,8-Hexahydro-5H-cyclopenta[b]naphtalèn-5-one

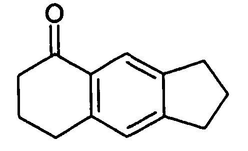

On refroidit à 0-5°C une suspension de 61 g (0,457 mole) de AlCl₃ anhydre dans 188 ml d'un mélange dichlorométhane/nitrométhane=8/1 et on y ajoute 22,15 g d'indane et, par portions, 22,5 g (0,225 mole) d'anhydride succinique. Après une heure à 0°C on verse dans un mélange eau/glace, on ajoute de l'acide chlorhydrique à 37% jusqu'à l'obtention d'une solution limpide. On extrait à l'acétate d'éthyle, on lave à l'eau on sèche la phase organique et on évapore le solvant sous pression réduite. On obtient un solide blanc qu'on dissout dans 500 ml de dioxane. On y ajoute 4,6 ml d'acide sulfurique à 98% dans 50 ml d'éthanol et 3,6 g de Pd/C à 10%. Après 5 heures en atmosphère d'hydrogène on filtre le catalyseur on évapore le solvant on reprend à l'eau et on extrait à l'acétate d'éthyle. On dissout le produit obtenu dans 20 ml de méthanol et on y ajoute 8 ml d'une solution aqueuse d'hydroxyde de sodium 1N et on agite à la température ambiante pendant deux heures. On évapore le solvant on ajoute de l'eau, on porte à pH acide et on extrait et on isole un solide blanc. On dissout 38,7 g de ce produit dans 1000 ml de chlorure de méthylène anhydre en atmosphère d'azote ; on refroidit à 0°C et on y ajoute avec précaution 47 g (0,225 mole) de PCl₃, on agite à 0°C pendant deux heures et après on y ajoute lentement 121,8 g (0,467 mole) de SnCl₄ et après 10 minutes on laisse chauffer jusqu'à la température ambiante. Après deux heures à la température ambiante on verse dans un mélange eau/glace, on extrait au chlorure de méthylène et on isole une huile qui est ensuite purifié par chromatographie sur colonne de gel de silice en éluant par un mélange cyclohexane/acétate d'éthyle = 8/2. On obtient 6,0 g du composé du titre.

### PREPARATION 2

### Acide 2,3-dihydro-1H-cyclopenta[b]naphtalèn-6-yl-acétique

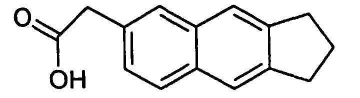

En opérant en courant d'azote on mélange 3 g d'hydrure de sodium à 80% (0,075 mole) dans 845 ml de tétrahydrofurane et on y ajoute 0,7 g (0,0375 mole) du produit de la Préparation 1 dissout dans 45 ml de tétrahydrofurane. Après 1 heure au reflux on ajoute 9 ml (0,075 mole) de diéthylcarbonate dans 45 ml de tétrahydrofurane et on laisse agiter au reflux pendant 5 heures. On refroidit le mélange on y ajoute une solution aqueuse saturée de chlorure d'ammonium, de l'eau jusqu'à dissolution complète et on extrait à l'acétate d'éthyle. On lave à l'eau on sèche la phase organique et on évapore le solvant sous pression réduite. On dissout l'huile obtenue dans 60 ml de tétrahydrofurane et on ajoute cette solution, à la température ambiante et en atmosphère d'azote, à une suspension d'hydrure de sodium à 80% (1,6 g ; 0,41 mole) dans 30 ml de tétrahydrofurane. On agite pendant 30 minutes on y ajoute 8,8 ml de bromoacétate d'éthyle (0,08 mole) et on agite à la température ambiante pendant 5 heures. On ajoute du chlorure d'ammonium et on extrait au chlorure de méthylène. On lave à l'eau on sèche la phase organique et on évapore le solvant sous pression réduite. On dissout l'huile ainsi obtenue dans 600 ml d'une solution hydroalcoolique d'hydroxyde de sodium 0,5 N (rapport EtOH/H₂O=2/1). On chauffe à environ 40°C jusqu'à la complète transformation du produit en son sel de sodium (formation d'un produit à la base dans l'essai de chromatographie sur couche mince en éluant par un mélange acétate d'éthyle/hexane=3/7. Après 8 heures environ, on évapore la moitié du solvant sous pression réduite, on acidifie avec 15 ml d'acide chlorhydrique 1 N et on chauffe à 50°C pendant 45 minutes environ. On extrait à l'acétate d'éthyle, on sèche la phase organique et on évapore le solvant sous pression réduite. On dissout le produit obtenu dans 60 ml d'acide acétique et on y ajoute 4,5 ml d'acide sulfurique à 98% dilué dans 9 ml d'acide acétique et 1 g de Pd/C à 10% ; ensuite on hydrogène pendant 8 heures. On évapore le solvant jusqu'à la moitié, on filtre le catalyseur on ajoute de l'eau et extrait à acétate d'éthyle, on sèche la phase organique et on évapore le solvant. On dissout le produit ainsi obtenu dans 100 ml de méthanol, on y ajoute 200 mg d'acide *para*-toluènesulfonique et on chauffe au reflux pendant 4 heures. On évapore partiellement le solvant on y ajoute 50 ml d'une solution aqueuse à 5% de bicarbonate de sodium on extrait à l'éther diéthylique on lave à l'eau et avec de la saumure. On sèche la phase organique, on évapore le solvant sous pression réduite. On dissout le produit ainsi obtenu dans de la décaline (20 ml), on y ajoute 1,4 g de Pd/C à 10% et on chauffe à 200°C pendant 48 heures. On filtre le catalyseur, on évapore le solvant et on purifie le brut de réaction par chromatographie sur colonne de gel de silice en éluant par un mélange cyclohexane/acétate d'éthyle=9/1. On dissout 750 mg du produit ainsi obtenu dan 30 ml d'éthanol, on y ajoute 20 ml de NaOH 1N et on chauffe à 50°C pendant 4 heures. On évapore l'éthanol et on acidifie avec de l'HCI 1N. On obtient un solide blanc.
P.f. 185-188°C.

### PREPARATION 3

### 5-(2-Bromoéthyl)-indane

On mélange 2 ml (0,016 mole) d'indane dans 33 ml de chlorure de méthylène et 1,6 ml (0,019 mole) de bromure de bromoacétyle à 0°C. On ajoute lentement 2,27 g (0,017 mole) d'AlCl₃ et on laisse revenir à la température ambiante et on agite pendant 2 heures. On verse le mélange dans de l'eau/glace et on extrait au chlorure de méthylène. On sèche la phase organique on filtre et on évapore le solvant sous pression réduite. On cristallise le brut de réaction dans de l'hexane. On sépare un solide blanc (P.f. 57,8-58,1°C) correspondant au produit d'acylation. On dissout 1,67 g (7 mmole) de ce produit dans 3,9 ml de triéthylsilane et 3,9 ml d'acide trifluoroacétique et on chauffe à 80°C pendant deux heures. On verse dans un mélange glace/NaOH et on extrait à l'acétate d'éthyle. On obtient le composé du titre.

### PREPARATION 4

### 2-(2-Bromoéthyl)-5,6,7,8,-tétrahydro-naphtalène

En opérant comme décrit dans la Préparation 3 mais en utilisant la tétraline au lieu de l'indane, on obtient le composé du titre.

### PREPARATION 5

### 5-(3-Bromopropyl)-indane

En opérant comme décrit dans la préparation 3 mais en utilisant le bromure de bromopropionyle au lieu du bromure de bromoacétyle, on obtient le composé du titre.

### PREPARATION 6

### 5-(3-Bromobutyl)-indane

En opérant comme décrit dans la préparation 3 mais en utilisant le bromure de bromobutanoyle au lieu du bromure de bromoacétyle, on obtient le composé du titre.

### PREPARATION 7

### 2-(2-Bromoéthyl)-fluorène

En opérant comme décrit dans la Préparation 3 mais en utilisant le fluorène au lieu de l'indane, on obtient le composé du titre.

### PREPARATION 8

### 2-(2-Bromoéthyl)-9,10-dihydrophénanthrène

En opérant comme décrit dans la Préparation 3 mais en utilisant le 9,10-dihydrophénanthrène au lieu de l'indane, on obtient le composé du titre.

### PREPARATION 9

### Acide 3-fluorènyl-acétique

### 9a) Acide 9-oxo-fluorèn-3-yl-carboxylique

On chauffe à 200°C un mélange de 0,45 g (0,00186 moles) d'acide 2,5-biphényl-dicarboxilique et 13,9 g d'acide polyphosphorique (PPA). Après une heure on refroidit à 100°C et on y ajoute de la glace jusqu'à obtenir un volume d'environ 100 ml. On filtre et on ajoute au précipité une solution de 0,5 g de NaOH dans 90 ml d'eau et on agite pendant une heure à 60°C. On acidifie avec de l'acide chlorhydrique et on extrait à l'acétate d'éthyle. On sèche la phase organique, on filtre et on évapore le solvant sous pression réduite. On obtient 0,31 g du produit du titre sous forme de solide marron clair.

### 9b) Acide fluorèn-3-yl-carboxylique

On dissout 0,27 g (0,0012 moles) du produit de l'étape précédente dans 2,5 ml de glycol éthylènique et on y ajoute 0,1 g de NaOH et 0,25 ml d'hydrazine à 98% (d=1,03). On chauffe au reflux pendant 1,5 heures on laisse revenir à la température ambiante, on ajoute 70 ml d'eau et on acidifie jusqu'à pH = 6 avec de l'acide chlorhydrique. On extrait avec del'acétate d'éthyle, on sèche la phase organique, on filtre et on évapore le solvant sous pression réduite. On obtient ainsi 0,4 g du produit du titre sous forme de solide jaune.

### 9c) Ester méthylique de l'acide fluorèn-3-yl-carboxylique

On prépare l'ester du produit de l'étape précédente à l'aide du méthanol dans de l'acide chlorhydrique. On porte à pH basique la solution et on extrait à l'acétate d'éthyle en obtenant ainsi le produit du titre sous forme d'huile.

### 9d) 3-Hydroxyméthyl-fluorène

On refroidit à 0°C et sous courant d'azote un mélange de 0,68 g de LiAlH₄ dans 7 ml d'éther éthylique anhydre et on y ajoute goutte à goutte une solution de 3,4 g (0,0152) du produit de l'étape précédente dans 27 ml d'éther éthylique anhydre et on agite pendant une nuit. On ajoute donc un mélange eau/glace, on extrait à l'acétate d'éthyle, on sèche la phase organique, on filtre et on évapore le solvant sous pression réduite. On obtient ainsi 3 g du produit brut qu'on purifie par chromatographie sur colonne de gel de silice en éluant par un mélange cyclohexane/acétate d'éthyle 7/3. On obtient ainsi 1,33 g du produit du titre sous forme de solide blanc.

### 9e) 3-Chlorométhyl-fluorène

On refroidit à 0°C une solution de 35 mg (0,178 mmoles) du produit de l'étape précédente dans 0,5 ml de chlorure de méthylène et on y ajoute goutte à goutte 0,13 ml de chlorure de thionyle (1,178 mmoles). On agite le mélange pendant trois heures à la température ambiante et on y ajoute un mélange d'eau et bicarbonate de sodium jusqu'à pH 8. On extrait au chlorure de méthylène on sèche la phase organique, on filtre et on évapore le solvant sous pression réduite. On obtient ainsi 20 mg du produit du titre sous forme d'huile jaune.

### 9f) 3-Cyanométhyl-fluorène

On dissout 1,3 g (0,00605 g) du produit de l'étape précédente dans 32 ml de DMSO et on y ajoute 0,44 g (0,00666 moles) de KCN. On chauffe à 80°C pendant 3 heures, on y ajoute un mélange eau/glace et on extrait à l'acétate d'éthyle. On sèche la phase organique, on filtre et on évapore le solvant sous pression réduite. On purifie le produit brut par flash-chromatographie en éluant par un mélange cyclohexane/acétate d'éthyle 8/2. On obtient ainsi 250 mg du produit du titre.

### 9g) Acide 3-fluorènyl-acétique

On hydrolise le cyano dérivé de l'étape précèdente à l'aide d'acide chlorhydrique 6N en chauffant au reflux. On ajoute un mélange eau/glace et on extrait à l'acétate d'éthyle. On sèche la phase organique, on filtre et on évapore le solvant sous pression réduite. On obtient ainsi 0,22 g du produit du titre sous forme de solide.
P.f.: 166-168°C

### PREPARATION 10

### Acide 2,3-dihydro-1H-cyclopenta[a]naphtalèn-8-yl-acétique

### 10a) 4,4-Diéthoxy-2-indan-4-yl-méthylèn-butyronitrile

On refroidit à 0°C sous courant d'azote 0,23 g (0,0092 moles) de NaH à 60% dans 20 ml de THF anhydre et on y ajoute une solution de 2,26 g (0,0085 moles) d'ester diéthylique de l'acide (1-cyano-3,3-diéthoxy-propyl)-phosphonique dans 16 ml de THF. Après 30 minutes le mélange devient limpide et on y ajoute alors goutte à goutte une solution de 1,1 g (0,0075 moles) de 2,3-dihydro-1H-indèn-8-yl-benzaldéhyde dans 16 ml de THF. On agite pendant une heure à 0°C et on ajoute un mélange d'eau/glace. On extrait au chlorure de méthylène, on lave la phase organique avec une solution de NaOH/eau, on sèche la phase organique, on filtre et on évapore le solvant sous pression réduite. On obtient ainsi 2,49 g du produit brut sous forme d'huile orange qu'on purifie par flash-cromatographie en éluant par un mélange cyclohexane/acétate d'éthyle 95/5. On obtient ainsi le produit du titre.

### 10b) 8-Cyano-2,3-dihydro-1H-cyclopenta[a]naphtalène

On refroidit à 0°C un mélange de 0,56 g (0,0021 moles) du produit de l'étape précédente dans 14 ml de chlorure de méthylène et on y ajoute goutte à goutte 152 ml (0,0013 moles) de SnCl₄ (d 2,226). On agite à la température ambiante pendant 4 heures, on y ajoute donc du bicarbonate de sodium à 10% et on extrait au chlorure de méthylène. On sèche la phase organique, on filtre et on évapore le solvant sous pression réduite. On obtient ainsi 0,43 g du produit du titre sous forme de solide jaune clair.

### 10c) 8-Méthylcarbonyl-2,3-dihydro-1H-cyclopenta[a]naphtalène

On dissout 0,5 g (0,0025 moles) du produit de l'étape précédente dans 20 ml de toluène anhydre et on y ajoute, goutte à goutte et sous courant d'azote, 1,6 ml (0,005 moles) de iodure de méthylmagnésium 3M. On agite pendant une nuit à la température ambiante, et on y ajoute donc de l'eau et de l'acide chlorhydrique. On agite pendant 15 minutes, on porte à pH basique avec une solution de NaOH 5M et on extrait à l'acétate d'éthyle. On lave la phase organique, on la sèche, on filtre et on évapore le solvant sous pression réduite. On obtient ainsi 0,69 g du produit brut qu'on purifie par flash-cromatographie en éluant par un mélange hexane/éther diéthylique 95/5. On obtient ainsi 0,35 g du produit du titre.

### 10d) 8-(Morpholino-thiocarbonyl)-2,3-dihydro-1H-cyclopenta

### [a]naphtalène

On dissout 0,35 g (0,0016 moles) du produit de l'étape précédente dans 0,35 ml (0,0016 mmoles) de morpholine (d 0,999) et on y ajoute 0,54 g (0,0020) de soufre et un crystal d'acide para-toluèn-sulphonique (PTSA). On chauffe au reflux pendant 6 heures et on ajoute donc du méthanol. On agite à la température ambiante pendant une nuit. On élimine le solvant sous pression réduite et on obtient ainsi 2,5 g du produit brut qu'on purifie par flash-cromatographie en éluant par un mélange cyclohexane/acétate d'éthyle 9/1. On obtient ainsi 0,16 g du produit du titre.

### 10e) Acide 2,3-dihydro-1H-cyclopenta[a]naphtalèn-8-yl-acétique

On dissout 0,16 g (0,0005 moles) du produit de l'étape précédente dans 7,6 ml d'une solution 1:1 de méthanol/eau et on y ajoute 0,02 g de NaOH solide. On chauffe au reflux pendant 6 heures, on élimine donc le solvant sous pression réduite, on reprend le résidu dans un mélange d'eau et acétate d'éthyle, on élimine la phase organique, on acidifie la phase aqueuse et on extrait au chlorure de méthylène. On sèche la phase organique, on filtre et on évapore le solvant sous pression réduite. On obtient ainsi 20 mg du produit du titre.

### PREPARATION 11

### Acide 5,6,7,8-tétrahydro-phénanthrèn-3-yl-acétique

En suivant le mode opératoire décrit dans la Préparation 10 mais en utilisant le 5,6,7,8-tétrahydro-naphtalèn-1-yl-benzaldéhyde au lieu du 2,3-dihydro-1H-indèn-7-yl-benzaldéhyde, on obtient le produit du titre.

### PREPARATION 12

### Acide 5,6,7,8-tétrahydro-anthracèn-2-yl-acétique

En suivant le mode opératoire décrit dans la Préparation 10 mais en utilisant le 5,6,7,8-tétrahydro-naphtalèn-2-yl-benzaldéhyde au lieu du 2,3-dihydro-1H-indèn-7-yl-benzaldéhyde, on obtient le produit du titre.

### EXEMPLE 1

### 1-[2-(2,3-Dihydro-1H-cyclopenta[b]naphtalèn-6-yl)-éthyl]-4-[3-trifluorométhyl-phényl]-1,2,3,6-tétrahydro-pyridine et son chlorhydrate

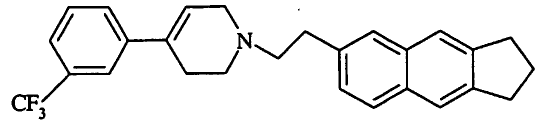

### 1a) 1-[4-Hydroxy-4-(3-trifluorométhyl-phényl)-1-pipéridinyl]-2-(2,3-dihydro-1H-cyclopenta[b]naphtalèn-6-yl)-1-éthanone

On dissout en atmosphère d'azote 400 mg (1,76 mole) du produit de la Préparation 2 dans 10 ml de chlorure de méthylène anhydre et on y ajoute 430 mg (1,76 mole) de 4-hydroxy-4-(3-trifluorométhyl-phényl)pipéridine, 0,79 g (1,76 mole) de BOP, 0,73 ml de triéthylamine et on agite à la température ambiante pendant 3 heures. On ajoute 40 ml d'acétate d'éthyle, on lave avec une solution d'acide chlorhydrique 1N, après avec une solution 1N d'hydroxyde de sodium et ensuite à l'eau. On sèche la phase organique sur du sulfate de sodium et on évapore le solvant. On obtient 0,8 g du composé du titre sous forme d'huile.

### 1b) 1-[2-(2,3-Dihydro-1H-cyclopenta[b]naphtalèn-6-yl)-éthyl]-4-hydroxy-4-(3-trifluoro-méthyl-phényl)-pipéridine

On dissout dans 7 ml de THF anhydre le produit obtenu par l'exemple la, on chauffe au reflux et y on ajoute 0,5 ml de diméthylsulfure de borane et on chauffe au reflux pendant 4 heures. On refroidit jusqu'à 0-5°C et on y ajoute avec précaution 7 ml de méthanol. Après 5 minutes on chauffe au reflux pendant 30 minutes, on évapore le solvant, on reprend le résidu dans le mélange eau/ammoniac=1/1, on extrait à l'acétate d'éthyle, on sépare les deux phases et on lave la phase organique à l'eau. On sèche sur du sulfate de sodium et on évapore le solvant sous pression réduite. On purifie le brut cristallisation dans de l'éther 2-propylique. On obtient 0,37 g du produit du titre.
P.f. 154-156° C.

### 1c) 1-[2-(2,3-Dihydro-1H-cyclopenta[b]naphtalèn-6-yl)-éthyl]-(4-(3-trifluorométhyl-phényl)-1,2,3,6-tétrahydro-pyridine et son chlorhydrate

On dissout 350 mg (0,8 mmole) du produit de l'étape précédente dans 10 ml d'acide acétique, on y ajoute 1 ml d'acide sulfurique à 96% et on chauffe à 80°C pendant 2 heures. On refroidit, on ajoute de l'NH₄OH concentré et on extrait à l'acétate d'éthyle. On lave à l'eau, on sèche et on évapore sous pression réduite. On obtient le composé du titre. On prépare le chlorhydrate à l'aide d'une solution de 2-propanol saturé en acide chlorhydrique.
P.f. (chlorhydrate) 264-266°C.

### EXEMPLE 2

### 1-[2-(2,3-Dihydro-1H-indèn-5-yl)-éthyl]-4-[3-trifluorométhyl-phényl]-1,2,3,6-tétrahydro-pyridine et son chlorhydrate

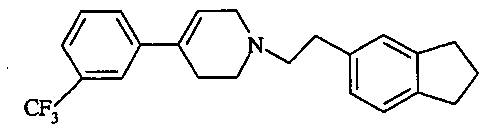

On dissout le produit obtenu dans la Préparation 3 dans 17 ml de butanol. On y ajoute 0,84 g (3,2 mmole) de 4-(3-trifluorométhyl-phényl)-1,2,3,6-tétrahydropyridine et 0,9 g (6,5 mmole) de carbonate de potassium et on chauffe au reflux pendant 5 heures. On évapore le solvant et on lave le résidu à l'eau. On extrait au chlorure de méthylène, on sèche la phase organique et on évapore le solvant sous pression réduite. On purifie le résidu sur colonne de gel de silice en éluant par un mélange cyclohexane/ acétate d'éthyle = 8/2. On obtient le composé du titre. On prépare le chlorhydrate à l'aide de 2-propanol saturé en acide chlorhydrique.
P.f. (chlorhydrate) 252-255°C.

### EXEMPLE 3

### 1-[2-(5,6,7,8-Tétrahydro-naphtalèn-2-yl)-éthyl]-4-[3-trifluorométhylphényl]-1,2,3,6-tétrahydro-pyridine et son chlorhydrate

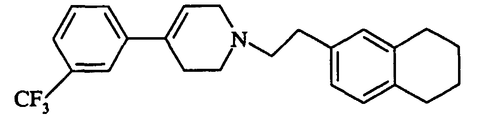

En opérant comme décrit dans l'Exemple 2 mais en utilisant le produit de la Préparation 4 au lieu du produit de la Préparation 3 on obtient les composés du titre.
P.f.(chlorhydrate): 263-267°C.

### EXEMPLE 4

### 1-[2-(2,3-Dihydro-1H-cyclopenta[b]naphtalèn-6-yl)-éthyl]-4-(6-chloropyrid-2-yl)-1,2,3,6-tétrahydropyridine et son chlorhydrate

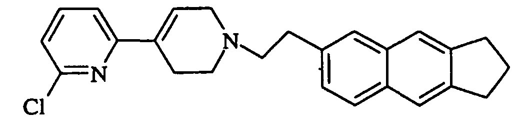

En opérant comme décrit dans l'Exemple 1 mais en utilisant la 4-(6-chloropyrid-2-yl)-1,2,3,6-tetrahydro-pyridine au lieu de la 4-(3-trifluorométhylphényl)-1,2,3,6-tétrahydro-pyridine, on obtient les composés du titre.
P.f.(chlorhydrate): 254-258°C.

### EXEMPLE 5

### 1-[2-(2,3-Dihydro-1H-cyclopenta[b]naphtalèn-6-yl)-éthyl]-4-(6-trifluorométhylpyrid-2-yl)-1,2,3,6-tétrahydropyridine et son chlorhydrate

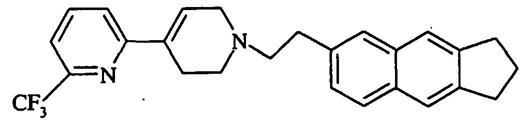

En opérant comme décrit dans l'Exemple 1 mais en utilisant la 4-(6-trifluorométhylpyrid-2-yl)-1,2,3,6-tétrahydro-pyridine au lieu de la 4-(3-trifluoromethyl-phényl)-1,2,3,6-tétrahydro-pyridine, on obtient les composés du titre.
P.f.(chlorhydrate): 264 -267°C.

### EXEMPLE 6

### 1-[3-(2,3-Dihydro-1H-indèn-5-yl)-propyl]-4-(3-trifluorométhyl-phényl)-1,2,3,6-tétrahydropyridine et son chlorhydrate

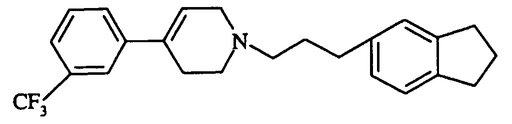

En opérant comme décrit dans l'Exemple 2 mais en utilisant le produit de la Préparation 5 au lieu du produit de la Préparation 3, on obtient les composés du titre.
P.f.(chlorhydrate): 192 -195°C.

### EXEMPLE 7

### 1-[4-(2,3-Dihydro-1H-indèn-5-yl)-butyl]-4-(3-trifluorométhyl-phényl)-1,2,3,6-tétrahydropyridine et son chlorhydrate

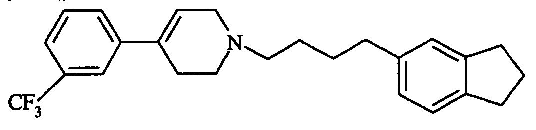

En opérant comme décrit dans l'Exemple 2 mais en utilisant le produit de la Préparation 6 au lieu du produit de la Préparation 3, on obtient les composés du titre.
P.f.(chlorhydrate): 198-200°C

### EXEMPLE 8

### 1-[2-(Fluorèn-2-yl)-éthyl]-4-(3-trifluorométhyl-phényl)-1,2,3,6-tétrahydropyridine et son chlorhydrate

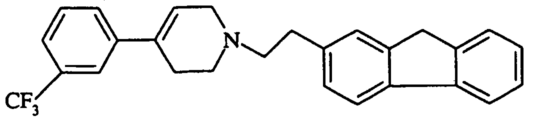

En opérant comme décrit dans l'Exemple 2 mais en utilisant le produit de la Préparation 7 au lieu du produit de la Préparation 3 on obtient les composés du titre.
P.f.(chlorhydrate): 285-287° C

### EXEMPLE 9

### 1-[2-(9,10-Dihydrophénanthrèn-2-yl)-éthyl]-4-(3-trifluorométhyl-phényl)-1,2,3,6-tétrahydropyridine et son chlorhydrate

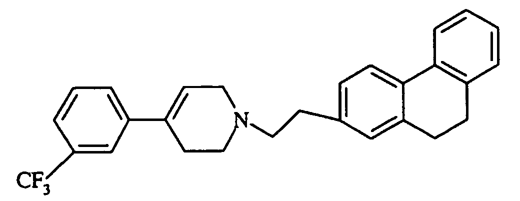

En opérant comme décrit dans l'Exemple 2 mais en utilisant le produit de la Préparation 8 au lieu du produit de la Préparation 3,on obtient les composés du titre.
P.f.(chlorhydrate): 256-258°C

### EXEMPLE 10

### 1-[2-(2,3-Dihydro-1H-cyclopenta[b]naphtalèn-6-yl)-éthyl]-(4-(3- trifluorométhyl-phényl)-1,2,3,6-tétrahydropyridine 1-oxyde

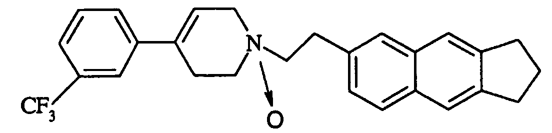

A une solution de 0,47 g (1,1 mmole) 1-[2-(2,3-dihydro-1H-cyclopenta[b]naphtalèn-6-yl)-éthyl]-4-(6-trifluorométhylpyrid-2-yl)-1,2,3,6-tétrahydropyridine dans 40 ml de chlorure de méthylène à la température de 0-5°C, on ajoute 0,27 g d'acide m-chloro-perbenzoïque. On laisse agiter à 0-5°C pendant deux heures, on lave avec une solution aqueuse saturée en bicarbonate de sodium et on sépare les deux phases. On sèche la phase organique, on filtre et on évapore sous pression réduite. On purifie par chromatographie en éluant par une mélange méthanol/acétate d'éthyle = 1/1 et on obtient le produit du titre.
P.f.: 116-117°C

### EXEMPLE 11

### 1-[2-(Fluorèn-3-yl)-éthyl]-(4-(3-trifluorométhyl-phényl)-1,2,3,6-tétrahydropyridine et son chlorhydrate

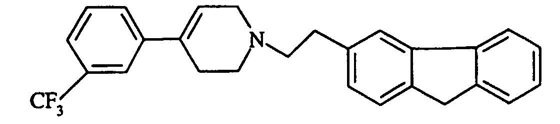

En opérant comme décrit dans l'Exemple 1 mais en utilisant le produit de la Préparation 9 au lieu du produit de la Préparation 2 on obtient les composés du titre.
P.f.(chlorhydrate): 238-240°C

### EXEMPLE 12

### 1-(2-(2,3-Dihydro-1H-cyclopenta[a]naphtalèn-8-yl)-éthyl]-4-(3-trifluorométhyl-phényl)-1,2,3,6-tétrahydropyridine et son chlorhydrate

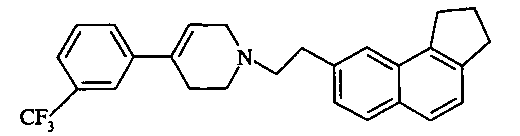

En opérant comme décrit dans l'Exemple 1 mais en utilisant le produit de la Préparation 10 au lieu du produit de la Préparation 2 on obtient les composés du titre.
P.f. (chlorhydrate): 212-213°C

### EXEMPLE 13

### 1-[2-(5,6,7,8-Tétrahydro-phénanthrèn-3-yl)-éthyl]-4-(3-trifluorométhylphényl)-1,2,3,6-tétrahydropyridine et son chlorhydrate

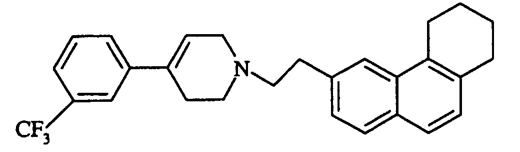

En opérant comme décrit dans l'Exemple 1 mais en utilisant le produit de la Préparation 11 au lieu du produit de la Préparation 2 on obtient les composés du titre.
P.f. (chlorhydrate): 222-224°C

### EXEMPLE 14

### 1-[2-(5,6,7,8-Tétrahydro-anthracèn-2-yl)-éthyl]-4-(3-trifluorométhylphényl)-1,2,3,6-tétrahydropyridine et son chlorhydrate

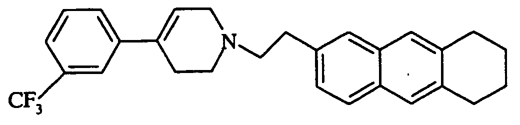

En opérant comme décrit dans l'Exemple 1 mais en utilisant le produit de la Préparation 12 au lieu du produit de la Préparation 2 on obtient les composés du titre.
P.f. (chlorhydrate): 252-253°C

## Revendications

1. Composé de formule (I): 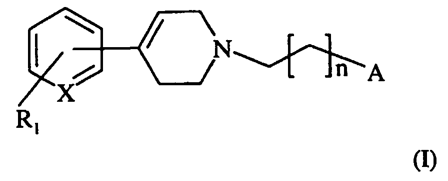 dans laquelle
X représente N ou CH ;
R₁ représente un atome d'hydrogène ou d'halogène ou un groupe CF₃;
n est en entier de 1 à 5 ;
A représente un groupe de formule (a) à (d): 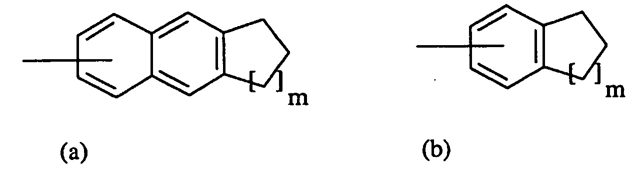 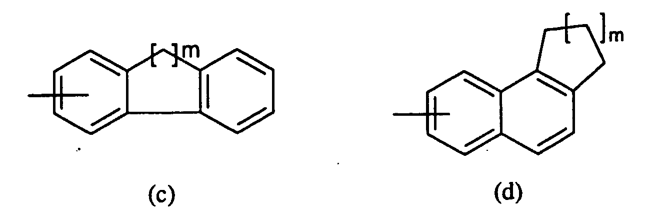 dans lesquelles m est 1 ou 2 ;
ainsi que ses sels ou solvates et leurs N-oxydes.

2. Composé selon la revendication 1 où n est 1.

3. Composé selon les revendications 1 ou 2 où R₁ est un groupe CF₃.

4. Composé selon les revendications 1 à 3 où X est CH et R₁ est dans la position 3 du benzène.

5. Composé selon les revendications de 1 à 3 où X est N et la pyridine est substituée dans les position 2,6.

6. Composé selon la revendication 1 choisi parmi les composés suivant:
1-[2-(2,3-dihydro-1H-cyclopenta[b]naphtalèn-6-yl)-éthyl]-(4-(3-trifluorométhyl-phényl)-1,2,3,6-tétrahydro-pyridine,
1-[2-(2,3-dihydro-1H-indèn-5-yl)-éthyl]-4-(3-trifluorométhyl-phényl]-1,2,3,6-tétrahydro-pyridine,
1-[2-(5,6,7,8-tétrahydro-naphtalèn-2-yl)-éthyl]-4-[3-trifluorométhylphényl]-1,2,3,6-tétrahydro-pyridine,
1-[2-(2,3-dihydro-1H-cyclopenta[b]naphtalèn-6-yl)-éthyl]-4-(6-chloropyrid-2-yl)-1,2,3,6-tétrahydropyridine,
1-[2-(2,3-dihydro-1H-cyclopenta[b]naphtalèn-6-yl)-éthyl]-4-(6-trifluorométhylpyrid-2-yl)-1,2,3,6-tétrahydropyridine,
1-[3-(2,3-dihydro-1H-indèn-5-yl)-propyl]-4-(3-trifluorométhyl-phényl)-1,2,3,6-tétrahydropyridine,
1-(4-(2,3-dihydro-1H-indèn-5-yl)-butyl]-4-(3-trifluorométhyl-phényl)-1,2,3,6-tétrahydropyridine,
1-[2-(fluorèn-2-yl)-éthyl]-4-(3-trifluorométhyl-phényl)-1,2,3,6-tétrahydropyridine
1-[2-(9,10-dihydrophénanthrèn-2-yl)-éthyl]-4-(3-trifluorométhyl-phényl)-1,2,3,6-tétrahydropyridine,
1-[2-(2,3-dihydro-1H-cyclopenta[b]naphtalèn-6-yl)-éthyl]-(4-(3-trifluorométhyl-phényl)-1,2,3,6-tétrahydropyridine,
1-[2-(fluorèn-3-yl)-éthyl]-(4-(3-trifluorométhyl-phényl)-1,2,3,6-tétrahydropyridine,
1-[2-(2,3-dihydro-1H-cyclopenta[a]naphtalèn-8-yl)-éthyl]-4-(3-trifluorométhyl-phényl)-1,2,3,6-tétrahydropyridine,
1-[2-(5,6,7,8-tétrahydro-phénanthrèn-3-yl)-éthyl]-4-(3-trifluorométhylphényl)-1,2,3,6-tétrahydropyridine,
1-[2-(5,6,7,8-tétrahydro-anthracèn-2-yl)-éthyl]-4-(3-trifluorométhylphényl)-1,2,3,6-tétrahydropyridine,
leurs sels et solvates et leurs N-oxydes.

7. Procédé de préparation d'un composé (I) de la revendication 1, **caractérisé en ce qu'**on
(a) fait réagir le composé de formule (II): 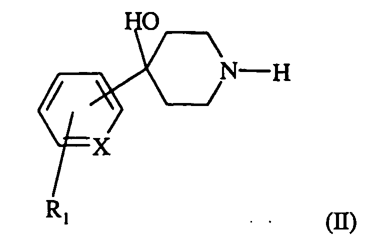 dans laquelle R₁ est défini dans la revendication 1, avec l'acide de formule (III) ou un de ses dérivés fonctionnels: 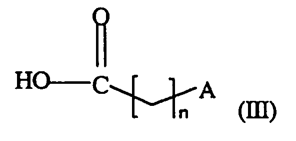 dans laquelle n et A sont tels que définis dans la revendication 1,
(b) on réduit le groupe carbonyle du composé de formule (IV) ainsi obtenu: 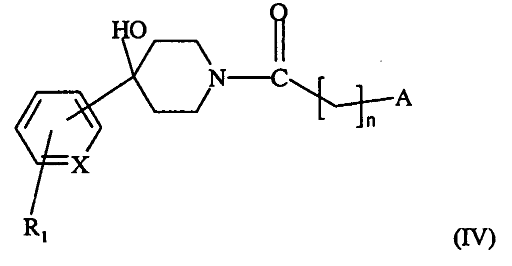
(c) on déshydrate le pipéridinol intermédiaire de formule (V): 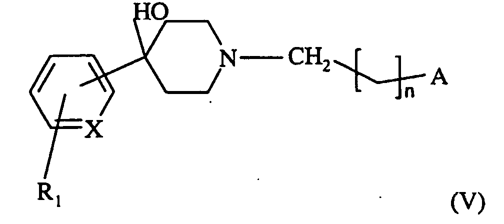
(d) on isole le composé de formule (I) ainsi obtenu et, éventuellement on le transforme en l'un de ses sels ou solvates ou leurs N-oxydes.

8. Composé de formule (III°) 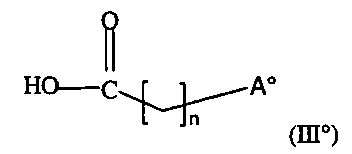 dans laquelle A° est un groupe de formule (a), (c) ou (d) comme définis dans la revendication 1, n est un entier de 1 à 5 et m est 1 ou 2, ainsi que ses sels ou solvates.

9. Composition pharmaceutique comprenant en tant que principe actif un composé de formule (I) ou l'un de ses sels, solvates ou N-oxydes pharmaceutiquement acceptables selon l'une des revendications 1 à 6.

10. Composition selon la revendication 9, **caractérisée en ce qu'**elle contient de 0,001 à 100 mg de principe actif.

11. Utilisation d'un composé de formule (I) ou d'un de ses sels, solvates ou N-oxydes pharmaceutiquement acceptables selon l'une des revendications 1 à 6 pour la préparation de médicaments analgésiques et/ou destinés au traitement des maladies liées à des troubles immunitaires et inflammatoires.

12. Médicament **caractérisé en ce qu'**il consiste en l'un des composés de formule (I) ou l'un de ses sels, solvates ou N-oxydes selon l'une des revendications 1 à 6.

## Patentansprüche

1. Verbindung der Formel (I) 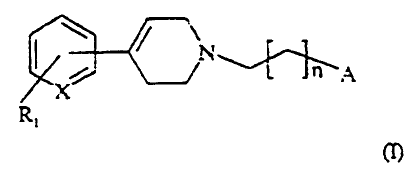 in der
X N oder CH darstellt;
R₁ ein Wasserstoffatom oder Halogenatom oder eine Gruppe CF₃ darstellt;
n ein Ganzes von 1 bis 5 ist;
A eine Gruppe der Formeln (a) bis (d) darstellt: 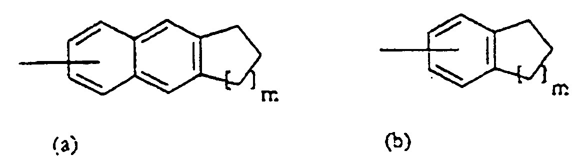 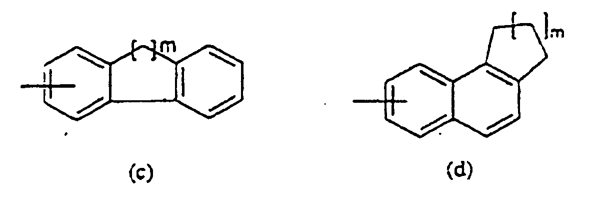 in denen m gleich 1 oder 2 ist;
sowie ihre Salze oder Solvate und ihre N-Oxide.

2. Verbindung nach Anspruch 1, worin n gleich 1 ist.

3. Verbindung nach Anspruch 1 oder 2, worin R₁ eine Gruppe CF₃ ist.

4. Verbindung nach Anspruch 1 bis 3, worin X CH ist und R₁ sich in Position 3 des Benzols befindet.

5. Verbindung nach Anspruch 1 bis 3, worin X N ist und das Pyridin in den Positionen 2,6 substituiert ist.

6. Verbindung nach Anspruch 1, ausgewählt unter den folgen Verbindungen:
1-[2-(2,3-Dihydro-1H-cyclopenta[b]-naphthalin-6-yl)-ethyl]-4-(3-trifluormethyl-phenyl)-1,2,3,6-tetrahydropyridin,
1-[2-(2,3-Dihydro-1H-inden-5-yl)-ethyl]-4-(3-trifluormethylphenyl)-1,2,3,6-tetrahydropyridin,
1-[2-(5,6,7,8-Tetrahydronaphthalin-2-yl)-ethyl]-4-(3-trifluormethyl-phenyl)-1,2,3,6-tetrahydropyridin,
1-[2-(2,3-Dihydro-1H-cyclopenta[b]-naphthalin-6-yl)-ethyl]-4-(6-chlorpyrid-2-yl)-1,2,3,6-tetrahydropyridin,
1-[2-(2,3-Dihydro-1H-cyclopenta[b]-naphthalin-6-yl)-ethyl]-4-(6-trifluormethylpyrid-2-yl)-1,2,3,6-tetrahydropyridin,
1-[2-(2,3-Dihydro-1H-inden-5-yl)-propyl]-4-(3-trifluormethylphenyl)-1,2,3,6-tetrahydropyridin,
1-[4-(2,3-Dihydro-1H-inden-5-yl)-butyl]-4-(3-trifluormethylphenyl)-1,2,3,6-tetrahydropyridin,
1-[2-(Fluoren-2-yl)-ethyl]-4-(3-trifluormethyl-phenyl)-1,2,3,6-tetrahydropyridin,
1-[2-(9,10-Dihydrophenanthren-2-yl)-ethyl]-4-(3-trifluormethylphenyl)-1,2,3,6-tetrahydropyridin,
1-[2-(2,3-Dihydro-1H-cyclopenta[b]-naphthalin-6-yl)-ethyl]-4-(3-trifluormethyl-phenyl)-1,2,3,6-tetrahydropyridin,
1-[2-(Fluoren-3-yl)-ethyl]-4-(3-trifluormethyl-phenyl)-1,2,3,6-tetrahydropyridin,
1-[2-(2,3-Dihydro-1H-cyclopenta[b]-naphthalin-8-yl)-ethyl]-4-(3-trifluormethyl-phenyl)-1,2,3,6-tetrahydropyridin,
1-[2-(5,6,7,8-Tetrahydrophenanthren-3-yl)-ethyl]-4-(3-trifluormethyl-phenyl)-1,2,3,6-tetrahydropyridin,
1-[2-(5,6,7,8-Tetrahydroanthracen-2-yl)-ethyl]-4-(3-trifluormethyl-phenyl)-1,2,3,6-tetrahydropyridin,
ihre Salze und Solvate und ihre N-Oxide.

7. Verfahren zur Herstellung einer Verbindung (I) von Anspruch 1, **dadurch gekennzeichnet, daß** man
(a) die Verbindung der Formel (II) 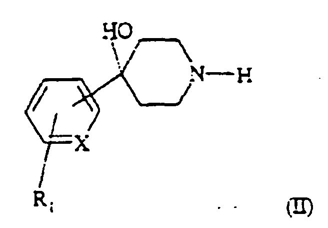 in der R₁ wie in Anspruch 1 definiert ist, mit der Säure der Formel (III) oder einem ihrer funktionellen Derivate 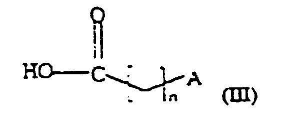 in der n und A wie in Anspruch 1 definiert sind, zur Reaktion bringt,
(b) die Carbonylgruppe der auf diese Weise erhaltenen Verbindung der Formel (IV) 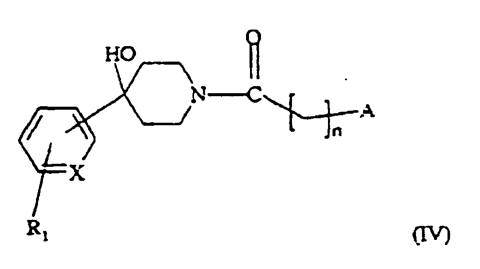 reduziert,
(c) das zwischenzeitlich erhaltene Piperidinol der Formel (V) 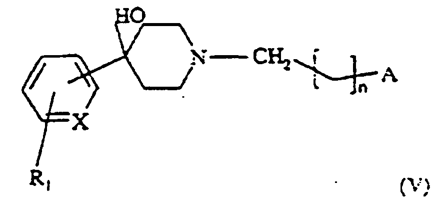 dehydratisiert,
(d) die auf diese Weise erhaltene Verbindung der Formel (I) isoliert und gegebenenfalls in eines ihrer Salze oder Solvate oder ihrer N-Oxide überführt.

8. Verbindung der Formel (III°) 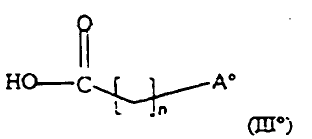 in der
A° eine Gruppe der Formel (a), (c) oder (d) ist wie in Anspruch 1 definiert, n ein Ganzes von 1 bis 5 ist und m gleich 1 oder 2 ist, sowie ihre Salze oder Solvate.

9. Pharmazeutische Zusammensetzung, umfassend als Wirkstoff eine Verbindung der Formel (I) oder eines ihrer pharmazeutisch akzeptablen Salze, Solvate oder N-Oxide nach einem der Ansprüche 1 bis 6.

10. Zusammensetzung nach Anspruch 9, **dadurch gekennzeichnet, daß** sie 0,001 mg bis 100 mg Wirkstoff enthält.

11. Verwendung einer Verbindung der Formel (I) oder eines ihrer pharmazeutisch akzeptablen Salze, Solvate oder N-Oxide nach einem der Ansprüche 1 bis 6 zur Herstellung von analgetischen Arzneimitteln und/oder vorgesehen zur Behandlung von Erkrankungen, die mit immunitären oder inflammatorischen Störungen verbunden sind.

12. Arzneimittel, **dadurch gekennzeichnet, daß** es aus einer der Verbindungen der Formel (I) oder einem ihrer Salze, Solvate oder N-Oxide nach einem der Ansprüche 1 bis 6 besteht.

## Claims

1. Compound of formula (I): 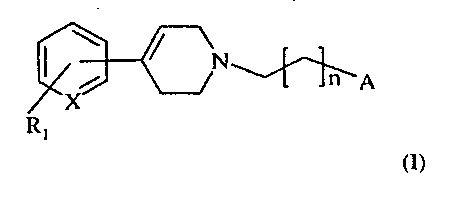 in which
X represents N or CH;
R₁ represents a hydrogen or halogen atom or a CF₃ group;
n is an integer from 1 to 5;
A represents a group of formula (a) to (d): 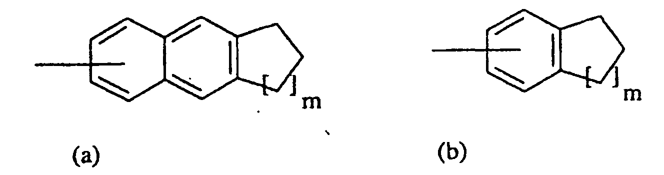 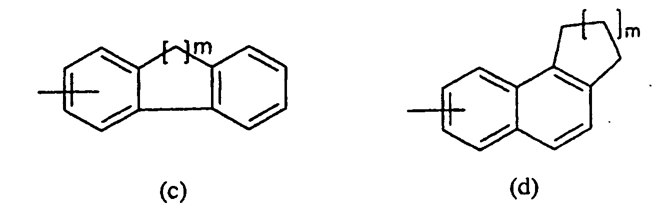 in which m is 1 or 2;
and its salts or solvates and its N-oxides.

2. Compound according to Claim 1, where n is 1.

3. Compound according to Claim 1 or 2, where R₁ is a CF₃ group.

4. Compound according to Claims 1 to 3, where X is CH and R₁ is at the 3-position of the benzene.

5. Compound according to Claims 1 to 3, where X is N and the pyridine is substituted at the 2,6-positions.

6. Compound according to Claim 1, chosen from the following compounds:
1-[2-(2,3-dihydro-1H-cyclopenta[b]naphthalen-6-yl)ethyl]-4-(3-trifluoromethylphenyl)-1,2,3,6-tetrahydropyridine,
1-[2-(2,3-dihydro-1H-inden-5-yl)ethyl]-4-(3-trifluoromethylphenyl]-1,2,3,6-tetrahydropyridine,
1-[2-(5,6,7,8-tetrahydronaphthalen-2-yl)ethyl]-4-[3-trifluoromethylphenyl]-1,2,3,6-tetrahydropyridine,
1-[2-(2,3-dihydro-1H-cyclopenta[b]naphthalen-6-yl)ethyl]-4-(6-chloropyrid-2-yl)-1,2,3,6-tetrahydropyridine,
1-[2-(2,3-dihydro-1H-cyclopenta[b]naphthalen-6-yl)ethyl]-4-(6-trifluoromethylpyrid-2-yl)-1,2,3,6-tetrahydropyridine,
1-[3-(2,3-dihydro-1H-inden-5-yl)propyl]-4-(3-trifluoromethylphenyl)-1,2,3,6-tetrahydropyridine,
1-[4-(2,3-dihydro-1H-inden-5-yl)butyl]-4-(3-trifluoromethylphenyl)-1,2,3,6-tetrahydropyridine,
1-[2-(fluoren-2-yl)ethyl]-4-(3-trifluoromethylphenyl)-1,2,3,6-tetrahydropyridine,
1-[2-(9,10-dihydrophenanthren-2-yl)ethyl]-4-(3-trifluoromethylphenyl)-1,2,3,6-tetrahydropyridine,
1-[2-(2,3-dihydro-1H-cyclopenta[b]naphthalen-6-yl)ethyl]-(4-(3-trifluoromethylphenyl)-1,2,3,6-tetrahydropyridine,
1-[2-(fluoren-3-yl)ethyl]-4-(3-trifluoromethylphenyl)-1,2,3,6-tetrahydropyridine,
1-[2-(2,3-dihydro-1H-cyclopenta[a]naphthalen-8-yl)ethyl]-4-(3-trifluoromethylphenyl)-1,2,3,6-tetrahydropyridine,
1-[2-(5,6,7,8-tetrahydrophenanthren-3-yl)ethyl]-4-(3-trifluoromethylphenyl)-1,2,3,6-tetrahydropyridine,
1-[2-(5,6,7,8-tetrahydroanthracen-2-yl)ethyl]-4-(3-trifluoromethylphenyl)-1,2,3,6-tetrahydropyridine,
their salts and solvates and their N-oxides.

7. Method for preparing a compound (I) of Claim 1, **characterized in that**
(a) the compound of formula (II): 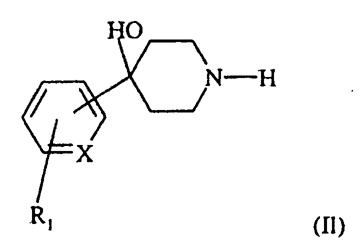 in which R₁ is as defined in Claim 1, is reacted with the acid of formula (III) or one of its functional derivatives: 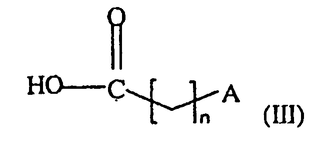 in which n and A are as defined in Claim 1,
(b) the carbonyl group of the compound of formula (IV) thus obtained: 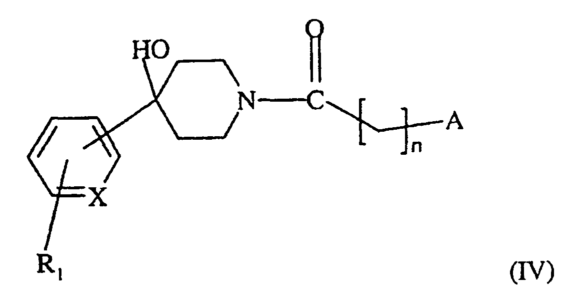 is reduced
(c) the piperidinol intermediate of formula (V): 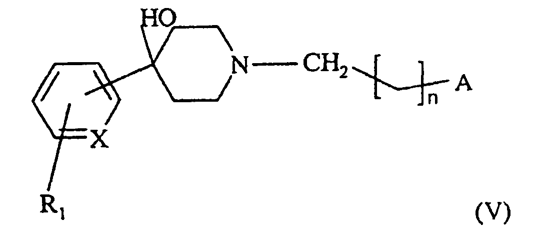 is dehydrated
(d) the compound of formula (I) thus obtained is isolated and it is optionally converted to one of its salts or solvates or its N-oxides.

8. Compound of formula (III°) 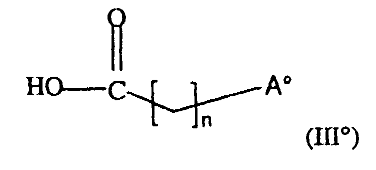 in which A° is a group of formula (a), (c) or (d) as Defined in claim 1, n is an integer from 1 to 5 and m is 1 or 2, and its salts or solvates.

9. Pharmaceutical composition comprising, as active ingredient, a compound of formula (I) or one of its pharmaceutically acceptable salts, solvates or N-oxides according to one of Claims 1 to 6.

10. Composition according to Claim 9, **characterized in that** it contains from 0.001 to 100 mg of active ingredient.

11. Use of a compound of formula (I) or of one of its pharmaceutically acceptable salts, solvates or N-oxides according to one of Claims 1 to 6 for the preparation of analgesic medicaments and/or medicaments intended for the treatment of diseases linked to immune and inflammatory disorders.

12. Medicament, **characterized in that** it consists of one of the compounds of formula (I) or one of its salts, solvates or N-oxides according to one of Claims 1 to 6.
